(19) Europäisches Patentamt
European Patent Office
Office européen des brevets

(11) EP 0 645 371 B1

(12) **EUROPÄISCHE PATENTSCHRIFT**

(45) Veröffentlichungstag und Bekanntmachung des Hinweises auf die Patenterteilung:
22.10.1997 Patentblatt 1997/43

(51) Int. Cl.$^6$: **C07C 227/40**, C07C 229/08

(21) Anmeldenummer: 94112748.2

(22) Anmeldetag: 16.08.1994

(54) **Verfahren zur Abtrennung von L-Leucin und L-Isoleucin aus wässrigen Lösungen**

Process for the separation of L-leucine and L-isoleucine from aqueous solutions

Procédé pour la séparation de L-leucine et L-isoleucine de solutions aqueuses

(84) Benannte Vertragsstaaten:
AT BE DE ES FR GB IT NL SE

(30) Priorität: 24.09.1993 DE 4332464

(43) Veröffentlichungstag der Anmeldung:
29.03.1995 Patentblatt 1995/13

(73) Patentinhaber: Degussa Aktiengesellschaft
60387 Frankfurt am Main (DE)

(72) Erfinder:
• Yonsel, Sems, Dr.
80660 Istinye-Istanbul (TR)
• Richet, Gérard
F-02100 Saint-Quentin (FR)
• Schäfer-Treffenfeldt, Wiltrud, Dr.
D-63179 Obertshausen 2 (DE)
• Le Quang, Tien
F-80400 Ham (FR)
• Sextl, Elfriede, Dr.
D-63826 Geiselbach (DE)
• Scholz, Mario, Dr.
D-63457 Hanau (DE)

(56) Entgegenhaltungen:
EP-A- 0 571 742        US-A- 4 910 336

• DATABASE WPI Week 6800, Derwent Publications Ltd., London, GB; AN 66-34753F & JP-B-43 020 181 (ASAHI CHEM IND CO LTD)
• PATENT ABSTRACTS OF JAPAN vol. 013, no. 056 (C-566) & JP-A-63 246 354 (AJINOMOTO CO INC)
• DATABASE WPI Week 9340, Derwent Publications Ltd., London, GB; AN 93-317422 & JP-A-05 229 995 (SUMITOMO CHEM CO LTD)

**Beschreibung**

Die Erfindung betrifft ein Verfahren zur Abtrennung von L-Leucin und L-Isoleucin aus wässrigen Lösungen und Trennung der Aminosäuren voneinander.

Leucin und Isoleucin sind Aminosäuren, die sich in der Anordnung der CH-Gruppen im neutralen Rest unterscheiden. Weitere Eigenschaften sind aus der Tabelle zu ersehen

|  | L-Leucin | L-Isoleucin |
|---|---|---|
| Molmasse [g/mol] | 131,17 | 131,17 |
| isoelektrischer Punkt | pH 5,98 | pH 5,94 |
| Löslichkeit [g/100 ml] | 2,19 | 4,12 |

Gemische, die diese beiden Aminosäuren enthalten, findet man z. B. in Proteinhydrolysaten. Wie aus dem Stand der Technik hervorgeht, gibt es erhebliche Schwierigkeiten, das L-Isoleucin von anderen verzweigten Aminosäuren, wie L-Leucin oder L-Valin zu trennen (Ullmann, Vol A2, Seite 70, 5th Edition, 1985).

Als Problemlösungen wurden z. B. die fraktionierte Kristallisation von Hydrochloriden (JP-PS 39-1915 (1964)) oder die Umwandlung der genannten Aminosäuren in das Kobaltsalz und die selektive Extraktion des entsprechenden L-Isoleucin-Salzes mit Alkoholen aus der Festphase ((JP-PS 37-15118 (1962), zitiert nach H. Samejima in "The Microbial Production of Amino Acids", Tokyo 1972, S. 245 und S. 253), angeboten.

Aus der deutschen Patentanmeldung P 42 17203.9 (europäische Patentanmeldung 93 105321.9) ist zwar bekannt, daß man Aminosäuren durch die Verwendung von Zeolithen aus wässrigen Lösungen abtrennen kann, es gibt jedoch keinen Hinweis auf die Trennung der eng verwandten Aminosäuren L-Leucin und L-Isoleucin voneinander.

Aufgabe der Erfindung ist es, ein einfacheres Trennverfahren für diese beiden Aminosäuren zur Verfügung zu stellen.

Gegenstand der Erfindung ist ein Verfahren zur Abtrennung von L-Leucin und L-Isoleucin aus diese Aminosäuren enthaltenden wässrigen Lösungen und ihre Trennung voneinander, das dadurch gekennzeichnet ist, daß man die wässrigen Lösungen gegebenenfalls mehrfach bei einem pH-Wert <7 mit einem Zeolith in Kontakt bringt, der einen Modul von 2 bis 1000, insbesondere 2 bis 200, besitzt, nach erfolgter Adsorption den Zeolith abtrennt und die adsorbierten Aminosäuren, deren Hauptanteil L-Leucin bildet, mit einer wässrigen, auf einen pH-Wert >7 eingestellten Lösung desorbiert.

Die Adsorption findet bevorzugt in einem pH-Bereich zwischen 1,0 und <7,0, bevorzugt 1 und 6 statt, wobei der pH-Wert der Lösungen z. B. mit Salzsäure oder Schwefelsäure eingestellt wird.

In einer Lösung mit einem Gemisch von Leucin und Isoleucin, bzw. weiteren neutralen Aminosäuren, ist die Adsorptionsbeladung von Leucin höher als die von Isoleucin. Es verbleibt somit eine Lösung mit einer im Verhältnis erhöhten Konzentration an Isoleucin, während das Leucin am Feststoff adsorbiert ist und leicht abgetrennt werden kann.

Die Desorption erfolgt durch Beaufschlagen des Zeoliths mit einer alkalischen Lösung, wie z. B. einer wässrigen Ammoniaklösung. Die Desorption findet im alkalischen Bereich, pH >7, bevorzugt bei pH 8 bis 11, statt.

Die Desorptionslösung enhält, abhängig vom eingestellten pH-Wert, im allgemeinen über 90 % der adsorbierten Leucinmenge.

Durch wiederholte Ad- und Desorptionszyklen, bzw. Optimierung des pH-Werts bei Ad- und Desorption, können Leucin und Isoleucin so getrennt werden, daß ausschließlich Leucin in der alkalischen Desorptionslösung und Isoleucin in der sauren Stammlösung vorkommen.

Das Verfahren findet in einem Temperaturbereich zwischen 15 und 60 °C statt, ohne daß die Kinetik wesentlich durch die Temperatur beeinflußt wird.

Im allgemeinen bewegt sich die Konzentration der Aminosäuren in den Lösungen bis zur jeweiligen Löslichkeitsgrenze.

Besondere Bedeutung besitzen jedoch Lösungen mit einem Gehalt von 10 bis 16 g/l L-Leucin und 4 bis 7 g L-Isoleucin.

Aus natürlichen Mineralien, wie Schweineborsten, Horn oder ähnlichem, gewonnene Hydrolysate enthalten nämlich immer wesentlich mehr L-Leucin als L-Isoleucin (Ullmann, 5th Edition, Amino Acids, Vol A2, Seite 57).

Bevorzugt eingesetzt werden Zeolithe der Typen A, X, insbesondere Y, DAY, Mordenit, dealuminierter Mordenit, ZSM-5, dealuminierter ZSM-5, Beta, VPI-5.

Besonders geeignet ist der ZSM-5 Typ in der H- oder Na-Form mit einem Modul von 2 bis 100 (Unter Modul versteht man das molare Verhältnis von $SiO_2$ zu $Al_2O_3$).

Je nach Bedarf werden die Zeolithe pulverförmig oder verformt eingesetzt.

Beispiele

Beispiel 1: Verwendung verschiedener Zeolithtypen

Folgende pulverförmige Zeolithtypen wurden eingesetzt, wobei die Kalzinierungsbedingungen keinen Ausschließlichkeitscharakter haben.

| | $SiO_2/Al_2O_3$ | Si/Al | Kalzinierung | |
|---|---|---|---|---|
| | | | [°C] | [h] |
| Mordenit | 30 | 15 | 550 | 1 |
| H-ZSM-5 | 45 | 23 | 550 | 1 |
| DAY | 200 | 100 | 950 | 1 |

Eine synthetische Lösung mit 15,2 g/l L-Leucin und 6.5 g/l L-Isoleucin wurde hergestellt und etwa 30 ml dieser Lösung in einem 100 ml Kolben mit etwa 3 g Zeolithpulver in Kontakt gebracht. Der pH-Wert wurde mit HCl auf pH 1 eingestellt und der Kolben etwa 20 h bei 21 °C geschüttelt. Nach der erfolgten Adsorption wurde der Überstand filtriert und analysiert.

Mit der Analyse der Aminosäurekonzentrationen vor ($C_o$) und nach ($C_f$) der Adsorption sowie Kenntnis der Adsorbenskonzentration ($C_z$)= g Zeolith/Aminosäurelösung wird die Beladung des Zeoliths bestimmt:

$$X = \frac{C_o - C_f}{C_z} \ [\%]$$

Folgende Adsorptionsbeladungen wurden erreicht:

| Zeolith | $X_{L-Leucin}$ (%) | $X_{L-Isoleucin}$ [%] |
|---|---|---|
| H-ZSM-5/M45 | 4,2 | 0,32 |
| Mordenit | 2,28 | 0,97 |
| DAY | 1,92 | 0,86 |

Beispiel 2: Trennung von L-Leucin und L-Isoleucin mit H-ZSM-5(M28)

Die Adsorptionsexperimente wurden in Schüttelkolben mit pulverförmigen Zeolithen durchgeführt. Der hier eingesetzte Zeolith, H-ZSM-5 hat ein Modul (Verhältnis von $SiO_2/Al_2O_3$) von 28 und wurde bei 550 °C 1 h kalziniert.

Eine Aminosäurelösung mit 15,6 g/l L-Leucin und 6,6 g/l L-Isoleucin wurde hergestellt. 89,5 ml dieser Lösung wurden mit 30,98 g pulverförmigem Zeolith H-ZSM-5, Modul 28, in einem 300 ml Kolben in Kontakt gebracht, und der pH-Wert mit HCl auf pH 2,5 gestellt.

Der Kolben wurde bei 21 °C über Nacht (etwa 20 h) geschüttelt, der Überstand filtriert und mit Hilfe einer HPLC-Anlage analysiert. Im Überstand lagen nach der Adsorption 0,3 g/l L-Leucin und 5,7 g/l L-Isoleucin vor.

Vor der Adsorption befanden sich in der Lösung von 89,5 ml insgesamt 1,99 g Aminosäuren, davon belief sich die Menge an L-Isoleucin auf 30 % und die von L-Leucin auf 70 %. Nach der Adsorption sind in der Lösung insgesamt 0,54 g Aminosäuren übriggeblieben.

L-Isoleucin hat dabei einen Anteil von 95 % und L-Leucin nur 5 %. Der Anteil von L-Isoleucin auf dem Zeolith belief sich auf 5 % (mit 0,07 g) und der von L- Leucin auf 95 % (mit 1,35 g).

Beispiel 3: Trennung von L-Leucin und L-Isoleucin aus einem Gemisch von weiteren neutralen Aminosäuren

400 ml einer Lösung enthielten neben 5,68 g Leucin, 3,44 g Isoleucin weitere Aminosäuren (Methionin, Valin, Alanin, Glycin, Phenylalanin) mit einem Mengenanteil von insgesamt 10 %, bezogen auf die Gesamtmenge der Aminosäuren.

Diese Lösung wurde über eine Säule mit 40,54 g Zeolith-Formkörpern (H-ZSM-5, Modul 45) geleitet. Der pH-Wert wurde mit HCl auf pH 1 eingestellt. Das Experiment lief bei Zimmertemperatur etwa 20 h. Im Überstand lagen nach der Adsorption 5,2 g Leucin und 3,24 g Isoleucin vor. Also wurden 0,48 g Leucin und 0,2 g Isoleucin an den Zeolithen adsorbiert. Die Beladungen beliefen sich auf 1,2 % für Leucin und 0,5 % für Isoleucin.

Die Säule wurde entleert, mit Wasser gespült und mit einer wässrigen Ammoniaklösung desorbiert. Der pH-Wert während der Desorption wurde auf pH 9,7 korrigiert.

Nach der Desorption wurden in der Lösung 0,44 g Leucin und 0,12 g Isoleucin wiedergefunden. Also konnten 92 % Leucin und 61 % Isoleucin vom Zeolith desorbiert werden.

Beispiel 4: Trennung von Leucin und Isoleucin mittels Adsorption und Desorption

Die Experimente wurden in Schüttelkolben bei Raumtemperatur mit pulverförmigen Zeolithen durchgeführt Zum Einsatz kamen: H-ZSM5 Modul 28 und H-ZSM5 Modul45.

| | | H-ZSM5 Modul 28 | H-ZSM5 Modul 45 |
|---|---|---|---|
| Konzentration in der Ausgangslösung: | L-LEU L-ILE | 10 g/l | 10 g/l |
| | | 10 g/l | 10 g/l |
| Verhältnis | LEU/ILE | 1:1 | 1:1 |
| Einwaage Zeolithpulver | | 23,9 g | 24,1 g |
| Einwaage Lösung | | 232,5 g | 252,5 g |
| Adsorption bei pH 5 | | | |
| Konzentration in der Lösung nach Adsorption | L-LEU L-ILE | 4,6 g/l | 6,5 g/l |
| | | 9,2 g/l | 9,6 g/l |
| Beladung des Zeolithen mit | L-LEU L-ILE | 1,25 g | 0,88 g |
| | | 0,18 g | 0,05 g |
| auf Masse des Zeolithen bezogene Beladung | L-LEU L-ILE | 5,25 % | 3,65 % |
| | | 0,78 % | 0,21 % |
| Verhältnis | LEU/ILE | 6,7:1 | 17,4:1 |
| Der beladene Zeolith wurde abgetrennt, mehrfach gewaschen und getrocknet. Danach wurde er für die Desorptionsexperimente eingesetzt. | | | |
| Einwaage Zeolithpulver | | 6,54 g | 6,03 g |
| Einwaage Desorptionslösung ($H_2O$) | | 62,7 g | 61,2 g |
| Desorption bei pH 10 | | | |
| Konzentration in der Desorptionslösung | L-LEU L-ILE | 5,25 g/l | 3,65 g/l |
| | | 0,65 g/l | 0,20 g/l |
| Desorbierte Menge | L-LEU L-ILE | 0,33 g | 0,22 g |
| | | 0,041 g | 0,012 g |
| Desorbierte Menge bezogen auf den Zeolithen: | L-LEU L-ILE | 5,05 % | 3,65 % |
| | | 0,68 % | 0,21 % |
| Verhältnis | LEU/ILE | 7,4:1 | 17,4:1 |

Die Versuche zeigen, daß über die Adsorption eine deutliche Aufkonzentrierung des Leucins erfolgt.

Während in der Ausgangslösung L-Leucin und L-Isoleucin in einem Mengenverhältnis von 1:1 enthalten sind, findet man nach dar Desorption in der desorbierten Flüssigkeit ein Verhältnis von 7,4 : 1 (Modul 28) bzw. sogar 17,4 : 1 (Modul 45).

Anders dargestellt bedeutet dies für die Aufreinigung des L-Leucin bei Verwendung des Typs ZSM5/M45 folgendes:

| Reinheit in der Ausgangslösung: | 50 %, |
|---|---|
| Reinheit nach 1. Trennschritt (Adsorption + Desorption): | 94,5 % |
| Reinheit nach 2. Trennschritt (Adsorption + Desorption) | 99,6 % |

## Patentansprüche

1. Verfahren zur Abtrennung von L-Leucin und L-Isoleucin aus diese Aminosäuren enthaltenden wässrigen Lösungen und ihreTrennung voneinander,
dadurch gekennzeichnet, daß man die wässrigen Lösungen gegebenfalls mehrfach bei einem pH-Wert <7 mit einem Zeolith in Kontakt bringt, der einen Modul von 2 bis 1000 besitzt, nach erfolgter Adsorption den Zeolith abtrennt und die adsorbierten Aminosäuren mit einer wässrigen, auf einen pH-Wert >7 eingestellten Lösung desorbiert.

2. Verfahren gemäß Anspruch 1,
dadurch gekennzeichnet, daß die Adsorption in einem pH-Bereich zwischen 1 und <7,0 erfolgt.

3. Verfahren gemäß den Ansprüchen 1 und 2,
dadurch gekennzeichnet, daß die Desorption in einem pH-Bereich zwischen 8 und 11 erfolgt.

4. Verfahren gemäß einem oder mehreren der Ansprüche 1 bis 3,
dadurch gekennzeichnet, daß man Zeolithe der Typen A, X, Y, DAY, Mordenit, dealuminierter Mordenit, ZSM-5, dealuminierter ZSM-5, Beta, VPI-5 einsetzt.

5. Verfahren gemäß Anspruch 4,
dadurch gekennzeichnet, daß man den ZSM-5-Typ in der H- oder Na-Form einsetzt.

6. Verfahren gemäß einem oder mehreren der Ansprüche 1 bis 5,
dadurch gekennzeichnet, daß die Lösung weitere neutrale Aminosäuren enthält.

## Claims

1. Process for the isolation of L-leucine and L-isoleucine from aqueous solutions containing these amino acids and their separation from each other, characterised in that the aqueous solutions are, optionally repeatedly, brought into contact at a pH of < 7 with a zeolite having a modulus of 2 to 1000, the zeolite is separated once adsorption is complete and the adsorbed amino acids are desorbed with an aqueous solution adjusted to a pH of > 7.

2. Process according to claim 1,
characterised in that adsorption is performed within a pH range of between 1 and < 7.0.

3. Process according to claims 1 and 2,
characterised in that desorption is performed within a pH range of between 8 and 11.

4. Process according to one or more of claims 1 to 3,
characterised in that the zeolites used are of the types A, X, Y, DAY, mordenite, dealuminated mordenite, ZSM-5, dealuminated ZSM-5, Beta, VPI-5.

5. Process according to claim 4,
characterised in that the ZSM-5 type is used in the H or Na form.

6. Process according to one or more of claims 1 to 5,
characterised in that the solution contains further neutral amino acids.

**Revendications**

1. Procédé pour la séparation de L-leucine et de L-isoleucine de solutions aqueuses contenant ces aminoacides, ainsi que pour leur séparation mutuelle, caractérisé en ce qu'on amène les solutions aqueuses éventuellement à plusieurs reprises à une valeur de pH < 7 en contact avec une zéolithe qui possède un module de 2 à 1000, on sépare la zéolithe une fois que l'adsorption a eu lieu et on soumet les amino-acides adsorbés à une désorption avec une solution aqueuse réglée à une valeur de pH > 7.

2. Procédé selon la revendication 1, caractérisé en ce qu'on effectue l'adsorption dans un domaine de pH entre 1 et < 7,0.

3. Procédé selon les revendications 1 et 2, caractérisé en ce qu'on effectue la désorption dans un domaine de pH entre 8 et 11.

4. Procédé selon l'une ou plusieurs des revendications 1 à 3, caractérisé en ce qu'on met en oeuvre des zéolithes des types A, X, Y, DAY, de la mordénite, de la mordénite désaluminée, ZSM-5, ZSM-5-désaluminée, bêta, VPI-5.

5. Procédé selon la revendication 4, caractérisé en ce qu'on met en oeuvre le type ZSM-5 dans la forme H ou Na.

6. Procédé selon l'une ou plusieurs des revendications 1 à 5, caractérisé en ce que la solution contient d'autres amino-acides neutres.